# EUROPÄISCHE PATENTANMELDUNG

(11) **EP 3 831 921 A1**
(43) Veröffentlichungstag der Anmeldung: **09.06.2021**
(21) Anmeldenummer: 20020590.4
(22) Anmeldetag: 04.12.2020
(51) Int. Cl.: C12M 1/107, C12M 1/00

(54) **BIOGASREAKTOR UND VERFAHREN ZU DESSEN BETRIEB SOWIE ENDFERMENTATIONSSTUFE**

(30) Priorität: 04.12.2019 DE 102019133040
(71) Anmelder: Hans Otto Mieth, 21481 Schnakenbek (DE)
(72) Erfinder: Hans Otto Mieth, 21481 Schnakenbek (DE)
(74) Vertreter: Völger, Karl Wolfgang

(57) **Zusammenfassung**

Die vorliegende Erfindung bezieht sich auf einen Biogasreaktor umfassend:
- einen zylindrischen Druckbehälter (1), der in seinem Innenraum zumindest eine Fermentationszone (101), einen Substratüberlauf (103) und eine Biogas-Sammelzone (105) aufweist,
- zumindest einen Vorratsbehälter (3) zur Bevorratung von Rohsubstrat,
- zumindest eine Rohsubstrat-Zufuhrleitung (5), die von dem Vorratsbehälter (3) in die Fermentationszone (101) des zylindrischen Druckbehälters (1) führt,
- zumindest eine Biogas-Entnahmeleitung (7) zum Abführen von entstandenem Biogas und
- zumindest einen Wertstoff-Sammelbehälter (9), der unterhalb des zylindrischen Druckbehälters (1) angeordnet und über eine Wertstoff-Sammelleitung (901) mit dem Überlaufrohr (103) verbunden ist,
wobei der Substratüberlauf (103a) des Überlaufrohrs (103) mindestens in der oberen Hälfte des zylindrischen Druckbehälters (1) positioniert ist.

Ferner bezieht sich die vorliegende Erfindung auf ein Verfahren zum Betreiben dieses Biogasreaktors.

Schließlich betrifft die vorliegende Erfindung eine Endfermentationsstufe für Biogasreaktoren und Biogasanlagen.

## Beschreibung

Die vorliegende Erfindung betrifft einen Biogasreaktor und ein Verfahren zu dessen Betrieb sowie eine Endfermentationsstufe, insbesondere ein Verfahren zur Erzeugung von Biogas und festen Wertstoffen.

Es ist aus dem Stand der Technik bekannt, organische Abfallstoffe aus der Tierhaltung und dem Ackerbau durch Fermentation in Biogas einerseits und in als Dünger einsetzbare Wertstoffe andererseits zu zerlegen. Das Biogas wird meist in angegliederten Gasmotoren mit Abgaswärmetauschern in Strom und Wärme (KWK) umgewandelt, wobei der Strom quantitativ zur Deckung des betrieblichen Bedarfes und/oder zur Einspeisung in das Versorgungsnetz genutzt wird. Die Wärme wird teilweise zur Beheizung des Fermentations-Prozesses und - soweit Bedarf besteht - zur Gebäudebeheizung sowie zur Warmwasser-Erzeugung eingesetzt.

Um eine Kostendegression einer großen Anlage gegenüber kleineren Anlagen zu nutzen, ist die Mehrzahl der praktisch ausgeführten Anlagen zentral angeordnet und besteht in der Regel aus ortsfesten kreisrunden, einige Meter hohen, betonierten Reaktoren mit zur Sammlung des entstehenden Biogases darüber gedeckten textilen Ballons.

Die Abfallstoff-Substrate werden vor Eintritt in den Reaktor mechanisch zerkleinert und je nach örtlichem/betrieblichem Aufkommen sowie unterschiedlichem Energiegehalt optimiert gemischt und so in den Reaktor eingespeist, dass der Fermentationsprozess in dessen gesamtem Inhalt gleichmäßig in Gang gehalten ist. Um aus dem Fermentations-Substrat als Dünger verwertbare Wertstoffe zu gewinnen, ist es erforderlich, dem Reaktor einen Abklingbereich nachzuschalten, in dem die Fermentation ohne Zufuhr neuen Substrates endet.

Zu den genannten großen Anlagen wurden Alternativkonzepte für dezentrale kleinere Anlagen vorgeschlagen. Zu nennen sind hier beispielsweise Containeranlagen, bei denen rechteckige Container als Reaktor und Nachklingbecken eingesetzt werden. Ferner wurden Anlagen mit liegendem zylindrischem Reaktor mit mechanischem Rühr- /Förderwerk für eine projektierte, aber noch nicht ausgeführte Biogasanlage eines Pferdehofes vorgeschlagen.

Die konventionellen Biogasanlagen nach dem Stand der Technik weisen noch weitere Nachteile auf. In einem dem Hauptfermenter nachgeschalteten Abklingbehälter findet in der Regel durch Rührwerke eine beständige Mischung des dort zur Restfermentation befindlichen Substrates mit im Takte der Hauptfermenter-Einspeisung von Rohsubstrat eingeleiteten teilfermentierten Substrat statt. Auf diese Art kann zwar im Abklingbehälter ein niedrigerer durchschnittlicher Gehalt an vergärbarer Substanz als im Hauptfermenter erreicht werden, der aber niemals gleich Null ist. Daher besteht die gesetzliche Vorschrift, das im Wesentlichen fermentierte Substrat für 180 Tage unter Luftabschluss zu lagern, um berechtigterweise die Umwelt vor frei austretendem Methan zu schützen. Diese vorgeschriebene 180-tägige Zwischenlagerung macht eine konventionelle Biogasanlage, selbst wenn sie so klein wie möglich dimensioniert ist, für die meisten potentiellen Anwender wirtschaftlich unrentabel und damit uninteressant.

Angesichts der im Stand der Technik bestehenden Problematik, dass es zwischen den großen Anlagen und mehr oder weniger improvisierten kleinen Anlagen keine gebrauchsfähigen Anlagen gibt, liegt der vorliegenden Erfindung daher die Aufgabe zugrunde, einen Biogasreaktor kleinerer Größe bereitzustellen und ein Verfahren zu dessen Betrieb anzugeben, mit welchen die Erzeugung von Biogas dezentral und in kleinerem Umfang ermöglicht wird. Darüber hinaus ist es Aufgabe der vorliegenden Erfindung, ausgehend von einem solchen Biogasreaktor eine Endfermentationsstufe bereitzustellen, mit der eine 180-tägige Zwischenlagerung vermieden und eine im Wesentliche kontinuierliche Substratgutabfuhr gewährleistet werden kann.

Diese Aufgabe wird in einem ersten Aspekt erfindungsgemäß durch einen Biogasreaktor gelöst, umfassend
- einen zylindrischen Druckbehälter (1), der in seinem Innenraum zumindest eine Fermentationszone (101), ein Überlaufrohr (103) und eine Biogas-Sammelzone (105) aufweist,
- zumindest einen Vorratsbehälter (3) zur Bevorratung von Rohsubstrat,
- zumindest eine Rohsubstrat-Zufuhrleitung (5), die von dem Vorratsbehälter (3) in die Fermentationszone (101) des zylindrischen Druckbehälters (1) führt,
- zumindest eine Biogas-Entnahmeleitung (7) zum Abführen von entstandenem Biogas und
- zumindest einen Wertstoff-Sammelbehälter (9), der unterhalb des zylindrischen Druckbehälters (1) angeordnet und über eine Wertstoff-Sammelleitung (901) mit dem Überlaufrohr (103) verbunden ist,
wobei der Substratüberlauf (103a) des Überlaufrohrs (103) mindestens in der oberen Hälfte des zylindrischen Druckbehälters (1) positioniert ist.

Der erfindungsgemäße Biogasreaktor weist die folgenden Vorteile gegenüber dem bekannten Stand der Technik auf:
- maximaler Verzicht auf Mechanik in der Fermentationszone (101), dadurch wartungsarm und energiesparend,
- durch systemimmanente "Trockenfermentation" optimaler Zustand des geernteten Wertstoffes (Dünger)
- minimaler Flächenbedarf des stehenden erfindungsgemäßen Biogasreaktors,
- Anpassung der Anlagenleistung durch Addition serienmäßig produzierter weiterer erfindungsgemäßer Biogasreaktoren,
- Werterhaltung des erfindungsgemäßen Biogasreaktors bei Schließung oder Verlagerung eines Betriebes durch Bemessung der Behälter auf verfügbare Transportmittel (LKW/Bahn),
- günstige Kostenstruktur durch Serienfertigung macht Dezentralisierung von Biogasanlagen in die Nähe der Abfallstoffproduktion und des Bedarfs für Abwärme wirtschaftlich und ökologisch wertvoll.

Der Biogasreaktor kann in einer speziellen Ausführungsform als ein stehender zylindrischer Druckbehälter (1) ausgeführt sein, der aus einem metallischen Werkstoff gefertigt ist. Insbesondere bevorzugt ist der zylindrische Druckbehälter (1) doppelwandig ausgeführt.

Das Überlaufrohr (103) dient neben dem Abführen des größtenteils ausfermentierten Substratguts auch zum Abklingen desselben und restlichen Fermentierten. Das Überlaufrohr (103) kann daher auch als Abklingrohr oder besser Restfermentationsrohr bezeichnet werden.

Es hat sich für das Abführen des entstehenden Wertstoffs, der insbesondere im Wesentlichen trocken vorliegt, als vorteilhaft herausgestellt, wenn der zylindrische Druckbehälter (1) am unteren Ende des Überlaufrohrs (103) einen konischen Auslauf (107) aufweist, der zumindest mit einer Wertstoff-Sammelleitung (901) in Verbindung steht.

Um das Fermentieren des Rohsubstrats zu verbessern und eine intensive Durchmischung von frischem Rohsubstrat und fast fertig ausfermentiertem Substratguts sicherzustellen, umfasst der erfindungsgemäße Biogasreaktor in einer speziellen Ausführungsform ferner eine Zirkulationsleitung (11) zum Zirkulieren von fermentierendem Substratgut aus einem oberen Bereich der Fermentationszone (101) in einen unteren Bereich der Fermentationszone (101).

Da ein Biogasreaktor von Zeit zu Zeit überholt und gereinigt werden muss, hat es sich als vorteilhaft herausgestellt, wenn dieser ferner umfasst
- eine Reinigungs-Vorlaufleitung (13), welche von der Rohsubstrat-Zufuhrleitung (5) in die Biogas-Sammelzone (105) führt, und
- zumindest eine Reinigungs-Rücklaufleitung (15), welche vom unteren Ende der Fermentationszone (101) in den Vorratsbehälter (3) führt.

Durch die Reinigungs-Vorlaufleitung (13) wird ein Reinigungsmedium von oben auf die Fermentationszone (101) aufgebracht und am unteren Ende durch die Reinigungs-Rücklaufleitung (15) wieder aufgefangen wird.

Ebenso vorteilhaft ist es für die Reinigung, wenn der erfindungsgemäße Biogasreaktor ferner zumindest eine Überlaufrohr-Rücklaufleitung (301) umfasst, welche vom unteren Ende des Überlaufrohrs (103) in den Vorratsbehälter (3) führt.

Da die Erfindung einen mittelgroßen bis kleinen Biogasreaktor zu Gegenstand hat, sind dessen Abmessungen in der praktischen Umsetzbarkeit entscheidend. Für eine gute Leistung hat es sich als sinnvoll herausgestellt, wenn das Verhältnis der Höhe des zylindrischen Druckbehälters (1) zu dessen Durchmesser bei 2 : 1 bis 7 : 1, bevorzugt bei 4 : 1 bis 6 : 1, liegt.

Bei einer aus praktischen Erwägungen sinnvollen Größe hat der erfindungsgemäße Biogasreaktor beispielsweise eine Höhe von 20 m und einen Durchmesser von 3,5 m.

Die vorstehend genannte Aufgabe wird in einem zweiten Aspekt der vorliegenden Erfindung durch ein Verfahren zum Betreiben eines Biogasreaktors gelöst, umfassend die Schritte
a) Erstbefüllen zumindest einer Fermentationszone (101) eines erfindungsgemäßen Biogasreaktors, wie er vorstehend beschrieben wurde, mit einem biologisch beimpften Rohsubstrat,
b) Fermentieren des Rohsubstrats in der Fermentationszone (101) über deren gesamte Höhe,
c) Abführen des ausfermentierten Substratguts über ein Überlaufrohr (103) und Auffangen in einem Wertstoff-Sammelbehälter (9),
d) Nachspeisen der Fermentationszone (101) mit Rohsubstrat in dem Umfang, in dem ausfermentiertes Substratgut abgeführt wird, und
e) Durchführen des weiteren Fermentierens zumindest in der oberen Hälfte der Fermentationszone (101).

Das erfindungsgemäße Verfahren weist im Wesentlichen die gleichen Vorteile wie der erfindungsgemäße Biogasreaktor auf.

In Schritt e) wird in einer bevorzugten Ausführungsform das Fermentieren in den oberen 2/3 der Füllhöhe der Fermentationszone (101) durchgeführt, insbesondere über die gesamte Füllhöhe der Fermentationszone (101).

Um das Fermentieren des Rohsubstrats zu verbessern und eine intensive Durchmischung von frischem Rohsubstrat und fast fertig ausfermentiertem Substratgut sicherzustellen, kann parallel zu den Schritten b), c) und d) fermentierendes Substratgut über eine Zirkulationsleitung (11) aus einem mittleren Bereich der Fermentationszone (101) in einen unteren Bereich der Fermentationszone (101) zirkuliert werden.

Worauf es erfindungsgemäß ankommt ist, dass der über die ganze Höhe der Fermentationszone (101) erfolgende Fermentationsprozess vor dem Eintritt in das Überlaufrohr (103) möglichst weitgehend abgeschlossen sein sollte. Da die Forderung besteht, aus der Wertstoff-Sammelleitung (901) nur vollständig fermentiertes Substratgut zu entnehmen, ist es sinnvoll, die Fermentationszone (101) im Durchmesser so reichlich auszulegen, dass das Überlaufrohr (103) mit möglichst weitgehend fermentiertem Substratgut beaufschlagt wird. Das optimale Verhältnis der Volumina der Fermentationszone (101) zum Überlaufrohr (103) mit der Endfermentation hängt von der Gärfreudigkeit der Rohsubstrate und der Effizienz der Maßnahmen und Zuschlagstoffe ab, die an den Rohsubstraten der jeweiligen landwirtschaftlichen Betriebe vor der Einspeisung in die Anlage vorgenommen werden.

Es ist bevorzugt, wenn das biologisch beimpfte Rohsubstrat eine zerkleinerte und breiig bis trockene Konsistenz aufweist. Auf diese Weise wird ein Wertstoff erhalten, welche selbst im Wesentlichen trocken ist und ohne größere Nachbehandlung eingesetzt werden kann.

Ein dritter Aspekt der vorliegenden Erfindung bezieht sich auf die Verwendung des erfindungsgemäßen Biogasreaktors, wie er vorstehend beschrieben wurde, als Endfermentationsstufe einer konventionellen Biogasanlage.

Wie vorstehend beschrieben wurde, wird das im Wesentliche ausfermentierte Substratgut durch das Überlaufrohr (103) (auch als Restfermentationsrohr zu bezeichnen) abgeführt, wo es durch die absinkende Verfahrensweise vollständig ausfermentiert wird. Dieses vollständige Ausfermentieren macht eine 180-tägige Lagerung überflüssig. Es hat sich daher als vorteilhaft erwiesen, den erfindungsgemäßen Biogasreaktor konventionellen Hauptfermerntern oder Nachfermentern nachzuschalten, um das zwar im Wesentlichen, aber doch nicht vollständig ausfermentierte Substratgut vollständig zu fermentieren.

Der Vorteil liegt darin, dass im Gegensatz zu den mehr als 5.000 Biogasanlagen allein in Deutschland, bei welchen durch die gesetzliche Auflage das weitgehend aber eben nicht vollständig ausfermentierte Restsubstrat aus dem oder den Haupt- und Nachgärbehältern 180 Tage unter Luftabschluss zu lagern ist, bevor es zur Düngung eingesetzt werden darf, der massive Kostendruck nicht vorhanden ist und die Nutzung der Restsubstrate als Dünger nicht prinzipiell infrage gestellt wird. Durch die erfindungsgemäße Verwendung des erfindungsgemäßen Biogasreaktors verliert die 180-tägige Lagerung ihren ökologischen Sinn. Die politisch immer wieder geforderte Kreislauf-Landwirtschaft wird wirtschaftlich, der Verbrauch an klimaschädlich erzeugtem Kunstdünger wird maßgeblich reduziert und die Energieausbeute des Biogasprozesses wird um den bei der 180-tägigen Lagerung verloren gehenden Teil an Methan (trotz des vorgeschriebenen aber praktisch nicht realisierbaren vollständigen Luftabschlusses) erhöht, indem das Gas in ein Biogaskraftwerk eingeleitet wird.

Die vorstehend genannte Aufgabe wird in einem vierten Aspekt der vorliegenden Erfindung durch eine Endfermentationsstufe für Biogasreaktoren und Biogasanlagen gelöst, umfassend
- einen zylindrischen Behälter (21), der in seinem Innenraum zumindest ein Überlaufrohr (2103) und eine Biogas-Sammelzone (2105) aufweist,
- einen den zylindrischen Behälter (21) nach oben abschließenden Deckel (2109),
- zumindest eine Zufuhrleitung (2111), die in den Innenraum des zylindrischen Behälters (21) zum oberen Ende des Überlaufrohrs (2103) führt,
- zumindest eine Biogas-Entnahmeleitung (27) zum Abführen von entstandenem Biogas und
- zumindest einen Wertstoff-Sammelbehälter (29), der unterhalb des zylindrischen Behälters (21) angeordnet und über eine Wertstoff-Sammelleitung (2901) mit dem Überlaufrohr (2103) verbunden ist,
wobei der zylindrische Behälter (21) vertikal angeordnet ist.

Die Definition, dass der zylindrische Behälter (21) vertikal angeordnet ist, ist so auszulegen, dass dieser aufrecht steht. Dabei kann der zylindrische Behälter (21) auch einen gewissen abweichenden Winkel gegenüber der absoluten Vertikalen einnehmen.

Ausgehend von dem vorstehend in Verbindung mit dem erfindungsgemäßen Biogasreaktor bereits beschriebenen Konzept ist in diesem Aspekt das Überlaufrohr (2103) explizit dazu vorgesehen, das im Wesentlichen ausfermentierte Substratgut, vielmehr die darin noch vorhandene vergärbare Restsubstanz vollständig abzubauen, bzw. auszufermentieren, so dass das am unteren Ende des Überlaufrohrs (2103) entnommene Substratgut sofort als Düngemittel eingesetzt werden kann, ohne dass die bei konventionellen Biogasanlagen zwecks Verhütung von Methanaustritt in die Atmosphäre vorgeschriebene 180-tägige Zwischenlagerung unter Luftabschluss eingehalten werden muss. Hierbei wird die vertikale Anordnung ausgenutzt, bei der das in dem Überlaufrohr (2103) von oben nach unten gleitende im Wesentlichen ausfermentierte Substratgut kontinuierlich gefördert und dabei kontinuierlich vollständig ausfermentiert wird.

Für den Betrieb der erfindungsgemäßen Endfermentationsstufe wird diese von oben über die zumindest eine Zufuhrleitung (2111) mit teilweise fermentiertem oder im Wesentlichen ausfermentiertem Substratgut beschickt, das aus Haupt- oder Nachfermentern einer konventionellen Biogasanlage zugeführt wird. Durch die Ausgestaltung des Überlaufrohrs (2103) als Endfermenter wird sichergestellt, dass es beim Absinken des aufgegebenen Substratguts nicht oder nur unwesentlich zu einer Vermischung der unterschiedlich weit ausfermentierten Substratgutanteile kommt. Dadurch wird verhindert, dass am Ende des Überlaufrohrs (2103) doch noch nicht vollständig ausfermentiertes Substratgut entnommen werden kann.

Das bei dieser Endfermentation zu vollständig ausfermentiertem Substratgut entstehende Biogas / Methan kann beispielsweise dem Gasspeicher der konventionellen Biogasanlage zugeführt und von dort beispielsweise einem BHKW zur Strom- und Wärmeerzeugung aufgegeben werden.

Die erfindungsgemäße Endfermentationsstufe weist verschiedenen Vorteile gegenüber dem Stand der Technik auf. Hier ist zuerst der Wegfall der gesetzlich vorgeschriebenen 180-tägigen Lagerung zwischen Anfall und Verwendung des Rest-Substratgutes aus konventionellen Biogasanlagen und der notwendigen Einrichtung (Lagerkapazitäten) dazu zu nennen. Ferner kann auch das bei der Endfermentation anfallende Biogas zusammen mit dem bei der Hauptfermentation gewonnenen Gas zur Energieerzeugung oder Aufbereitung zur Einspeisung ins vorhandene Erdgasnetz genutzt werden, was bei der 180-tägigen Lagerung nicht oder nur mit sehr hohem (und damit unwirtschaftlichem) Aufwand möglich ist. Schließlich ist es durch den einfachen, aber effizienten Aufbau der erfindungsgemäßen Endfermentationsstufe möglich, auf eine kostengünstige Bauweise und kostengünstige Fertigteile zurückzugreifen, z.B. aus gestapelten Betonringen großen Durchmessers mit metallischem Oberboden und spezialgefertigtem Auslaufkonus. Zudem kann beim Neubau von konventionellen Biogasanlagen der konventionelle Abklingbehälter oder Nachfermenter entfallen, der Flächenbedarf für eine neugebaute konventionelle Biogasanlage sinkt deutlich. Schließlich kann die erfindungsgemäße Endfermentationsstufe ohne großen technischen Aufwand bei bestehenden konventionellen Biogasanlagen nachgerüstet werden.

Durch die mit der erfindungsgemäßen Endfermentationsstufe mögliche Verfahrensweise ist zudem sichergestellt, dass das zuerst aufgegebene Substratgut auch zuerst wieder entnommen wird (sog. "first in - first out" Prinzip)

In einer Weiterbildung der erfindungsgemäßen Endfermentationsstufe weist der zylindrische Behälter (21) am unteren Ende des Überlaufrohrs (2103) einen konischen Auslauf (2107) auf, der zumindest mit einer Wertstoff-Sammelleitung (2901) in Verbindung steht. Der Konus hat sich für das Abführen des entstehenden Wertstoffs, der insbesondere im Wesentlichen trocken vorliegt, als vorteilhaft herausgestellt.

Eine Ausführungsform der erfindungsgemäßen Endfermentationsstufe sieht vor, dass das Überlaufrohr (2103) in vertikaler Richtung in zumindest zwei Teilzellen (2103a, 2103b) unterteilt ist.

Wesentlich an diesen Merkmalen ist die in vertikaler Richtung, also in Richtung des Überlaufrohrs (2103) schlanke Gestalt der zumindest zwei Teilzellen (2103a, 2103b), die oben gegenüber der Atmosphäre geschlossen und unten mit der Wertstoff-Sammelleitung (2901) verbunden sind.

Die in vertikaler Richtung vorgesehene schlanke Gestalt der zumindest zwei Teilzellen (2103a, 2103b) stellt sicher, dass es zwischen dem oben aufgegebenen Substratgut, dem in der Mitte des Überlaufrohrs (2103) befindlichen Substratgut und dem unten abzuziehenden Substratgut keine oder zumindest nur eine unwesentliche (vertikale) Vermischung beim Absinken der Substratgutchargen gibt.

Die vorliegende Ausführungsform kann praktisch in verschiedenen Varianten ausgebildet sein.

So kann das Überlaufrohr (2103) durch innenliegende konzentrische Ringe in die Teilzellen (2103a, 2103b, ...) unterteilt werden, wodurch ein vertikales Vermischen der fermentierenden Substratgutchargen unterbunden wird. Der als eine Biogas-Sammelzone (2105) ausgebildete Kopfraum und der unten abschließende Substratgutentnahmekonus sind den Zellen gemeinsam.

Ferner kann anstelle eines großvolumigen, durch konzentrische Ringe unterteilten Überlaufrohrs (2103) ein Bündel im Durchmesser optimaler Rohre Teilzellen (2103a, 2103b, ...) auf einer gemeinsamen Plattform vorgesehen sein.

Darüber hinaus müssen die Teile der erfindungsgemäßen Endfermentationsstufe nicht aus Metall / Stahl gefertigt sein, es kann auch ein Behälter aus Betonfertigteilen hergestellt werden, der nach dem geometrischen Konzept der vorliegenden Erfindung ausgelegt ist. Die Geometrie ist nicht auf runde oder ovale Formen begrenzt, es können auch mehreckige Grundformen Einsatz finden.

Im Rahmen der vorliegenden Erfindung kann das aus einer konventionellen Biogasanlage entnommene zumindest größtenteils fermentierte Substratgut auf die Erfordernisse der erfindungsgemäßen Endfermentationsstufe konditioniert werden. Hierdurch kann die Durchsatzleistung der erfindungsgemäßen Endfermentationsstufe maximiert und die vollständige Fermentation bezüglich der vergärbaren Restsubstanz optimiert werden. Dabei wird ein Substratgut erhalten, dessen Anteil an vergärbare Restsubstanz praktisch gleich Null ist, so dass bei deren sofortiger Ausbringung als Dünger kein klimaschädliches Methan gebildet wird. Es kann also auf die 180-tägige Zwischenlagerung unter Luftabschluss verzichtet werden.

Für diese genannte Konditionierung können erfindungsgemäß verschiedene Vorrichtungen vorgesehen und Vorkehrungen getroffen werden.

Zunächst kann der Flüssigkeitsgehalt des Substratguts zur Gewährleistung des sog. "first in - first out" Prinzips durch Trocknung oder durch Befeuchtung so standardisiert werden, dass einerseits das Abwärtsgleiten im Rohrquerschnitt gewährleistet ist, aber andererseits eine vertikale Mischbewegung (sog. "Kochen") der Schichten der Substratgutchargen vermieden wird.

In einer anderen Weiterbildung der erfindungsgemäßen Endfermentationsstufe ist am Eintritt in den zylindrischen Behälter (21) ein Durchlaufheizregister zur Temperierung von fermentierendem Substratgut vorgesehen.

Da die Temperatur des Substratguts für eine vollständige Fermentation sehr wichtig ist, wird mit dem Durchlaufheizregister, vorzugsweise an oder in der Zufuhrleitung (2111) die Temperatur standardisiert eingestellt, die sich bei Vorversuchen als vorteilhaft für die Maximierung der Durchsatzleistung der erfindungsgemäßen Endfermentationsstufe erwiesen hat.

Eine spezielle Weiterbildung sieht vor, dass die für den Betrieb des Durchlaufheizregisters benötigte Energie aus dem der gesamten Anlage nachgelagerten BHKW stammt.

Eine andere erfindungsgemäße Ausführungsform sieht zusätzlich die Homogenisierung des Substratguts vor. Beim Einsatz hinter konventionellen Biogasanlagen ist bei schwankender Zusammensetzung der Rohsubstrate mit chargenweise variierender für den Betrieb der erfindungsgemäßen Endvergärungsstufe periodisch suboptimaler Konsistenz des zugeführten, zumindest größtenteils fermentierten Substratguts zu rechnen. Es wird daher ein zwischengeschaltetes Aggregat vorgesehen, das durch Zerkleinern der Feststoffanteile in den einzelnen Substratgutchargen und deren intensive Durchmischung mit dem Flüssigkeitsanteil den in anlagenspezifischen Vorversuchen ermittelten, für die Funktion der erfindungsgemäßen Endfermentationsstufe optimalen Aggregatzustand für die permanente Einhaltung des "first in - first out" Prinzips bei maximierter Fermentationsleistung herbeiführt.

Es kann erfindungsgemäß ferner eine Einrichtung zur Beimpfung des zumindest größtenteils fermentierten Substratguts vorgesehen werden. Hierbei wird mit mikrobiell angereichertem Substratgut zur Auffrischung des Fermentationsprozesses in dem aus der Haupt- oder Abklingstufe konventioneller Biogasanlagen zugeführten Substratguts beimpft.

Alternativ kann eine Beimpfung mit mit katalytischen Elementen angereichertem Substratgut zur Beschleunigung des Fermentationsprozesses in dem aus der Haupt- oder Abklingstufe konventioneller Biogasanlagen zugeführten Substratguts vorgenommen werden. Da diese katalytischen Stoffe im ausfermentierten, für die Gründüngung bestimmten Substratgut verbleiben, kommen dafür nur katalytische Wirkstoffe infrage, die für das Pflanzenwachstum verträglich und beim Verbleib im Ackerboden und Grundwasser unschädlich sind.

Eine Weiterbildung der Erfindung sieht vor, dass in dem Überlaufrohr (2103) katalytische Elemente vorgesehen sind.

Konkret können zur Unterstützung der Endvergärung, bzw. vollständigen Fermentation den Fermentationsprozess unterstützende katalytische Bauelemente in die Substratgutführenden Bauelemente, insbesondere in das Überlaufrohr (2103) oder die Teilzellen (2103a, 2103b, ...) eingebaut werden. Die stoffliche Beschaffenheit dieser katalytischen Bauelemente muss nicht zwingend den strengen Anforderungen für die vorstehend genannten katalytischen Stoffe erfüllen, da diese nicht in das für die Gründüngung bestimmte Substratgut übergehen.

Die katalytischen Elemente können als maximal über die gesamte Höhe des Überlaufrohrs (2103) hängende, durch den oberen Boden eingeführte, an von außen lösbaren Flanschen montierte herausziehbare Träger ausgeführt sein, die vorzugsweise zwecks maximalen Kontaktes mit dem vorbeigleitenden Substratgut als sieb- oder fächerartige Strukturen gestaltet sind

Bei nachlassender katalytischer Wirkung der katalytischen Elemente können diese entweder im Einbauzustand regeneriert oder gegen neue bzw. extern regenerierte katalytische Elemente ausgetauscht werden. Es ist besonders bevorzugt, wenn die Regenerierung und/oder der Austausch ohne Stilllegung oder Totalentleerung der erfindungsgemäßen Endfermentationsstufe vorgesehen werden

Für die Vermeidung der Rückvermischung verschiedener Substratgutchargen hat es sich als vorteilhaft herausgestellt, wenn das Verhältnis der Höhe des Überlaufrohrs (2103) oder der Höhe jedes der zumindest zwei Teilzellen (2103.1, 2103.2) zu deren Durchmesser bei 2 : 1 bis 7 : 1, bevorzugt bei 4 : 1 bis 6 : 1, liegt.

Weitere Ziele, Merkmale, Vorteile und Anwendungsmöglichkeiten ergeben sich aus der nachfolgenden Beschreibung von die Erfindung nicht einschränkenden Ausführungsbeispielen anhand der Figuren. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbeziehung. Es zeigen:
- Fig. 1:: eine schematische Ansicht eines erfindungsgemäßen Biogasreaktors nach einer Ausführungsform der Erfindung,
- Fig. 2:: eine schematische Ansicht einer erfindungsgemäßen Endfermentationsstufe nach einer Ausführungsform der Erfindung und
- Fig. 3: eine schematische Detailansicht einer erfindungsgemäßen Endfermentationsstufe nach einer anderen Ausführungsform der Erfindung.

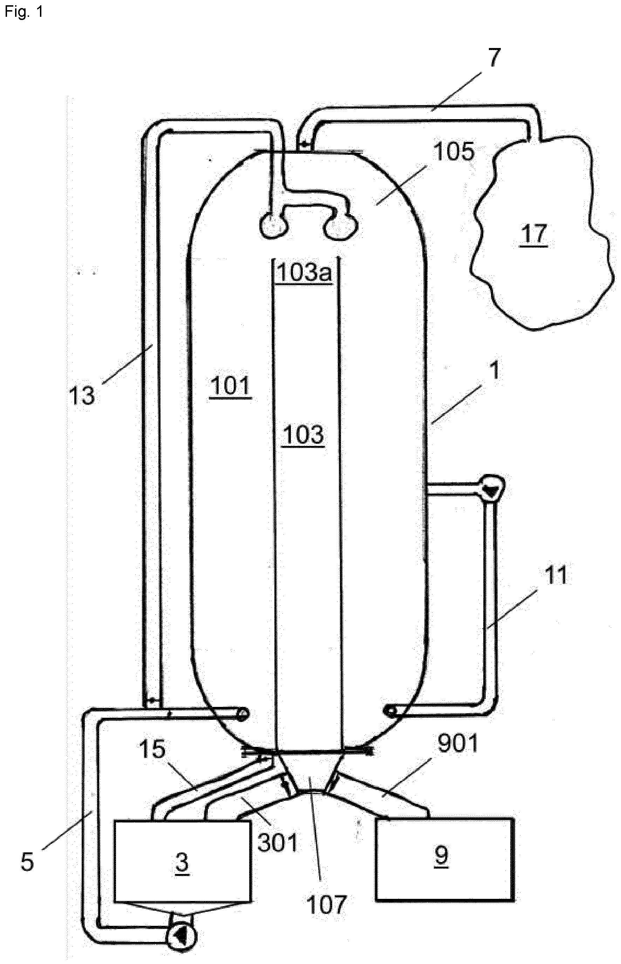

In Figur 1 wird der erfindungsgemäße Biogasreaktor in einer bevorzugten Ausführungsform dargestellt. Der zylindrische Druckbehälter 1 weist in seinem Innenraum zumindest eine Fermentationszone 101, ein Überlaufrohr 103 und eine Biogas-Sammelzone 105 auf. Unterhalb ist zumindest ein Vorratsbehälter 3 zur Bevorratung von Rohsubstrat anordnet, in dem dieses auch gemischt, eingewogen, zerkleinert und/oder homogenisiert werden kann.

Zumindest eine Rohsubstrat-Zufuhrleitung 5 führt von dem Vorratsbehälter 3 in die Fermentationszone 101 des zylindrischen Druckbehälters 1, vorzugsweise weit unten in der Nähe des Bodens des zylindrischen Druckbehälters 1. Diese Leitung wird insbesondere durch eine Pumpe unterstützt.

Zumindest eine Biogas-Entnahmeleitung 7 zum Abführen von entstandenem Biogas ist am oberen Ende des zylindrischen Druckbehälters 1 vorgesehen und führt in einen Biogas-Sammelbehälter 17. Zwischen der Biogas-Sammelzone 105 und der Biogas-Entnahmeleitung 7 ist vorteilhafterweise ein Absperrventil angeordnet.

Zumindest ein Wertstoff-Sammelbehälter 9 ist unterhalb des zylindrischen Druckbehälters 1 angeordnet und über eine Wertstoff-Sammelleitung 901 mit dem Überlaufrohr 103 verbunden. Auch hier kann ein Absperrventil vorgesehen sein, um beispielsweise bei einer Reinigung oder bei einer Entlüftung bei Inbetriebnahme dienlich zu sein.

Es ist erfindungsgemäß besonders bevorzugt, wenn der Substratüberlauf 103a des Überlaufrohrs 103 mindestens in der oberen Hälfte des zylindrischen Druckbehälters 1 positioniert ist, vorteilhafter Weise im oberen Drittel, insbesondere im oberen Viertel.

Wie aus der Figur 1 zu entnehmen ist, weist der zylindrische Druckbehälter 1 am unteren Ende des Überlaufrohrs 103 einen konischen Auslauf 107 auf, der zumindest mit der Wertstoff-Sammelleitung 901 in Verbindung steht. Ein Absperrventil kann am Übergang von konischem Auslauf 107 zu Wertstoff-Sammelleitung 901 vorgesehen sein.

Eine Zirkulationsleitung 11 zum Zirkulieren von fermentierendem Substratgut aus einem oberen Bereich der Fermentationszone 101 in einen unteren Bereich der Fermentationszone 101 unterstützt die Fermentation. Dabei kann vorteilhafterweise eine Pumpe eingesetzt werden, so dass heftig fermentierendes Substratgut in das frisch aus dem Vorratsbehälter 3 in die Fermentationszone 101 eingebrachte Rohsubstrat über eine mit einer Düse für das Rohsubstrat gleichgerichteten, hohe Turbulenz erzeigenden Tangentialdüse gemischt wird.

Eine Reinigungs-Vorlaufleitung 13, welche von der Rohsubstrat-Zufuhrleitung 5 (vorteilhaft mit einem Absperrventil versehen) in die Biogas-Sammelzone 105 führt, ist ebenso vorgesehen wie zumindest eine Reinigungs-Rücklaufleitung 15, welche vom unteren Ende der Fermentationszone 101(ebenfalls vorteilhaft mit einem Absperrventil versehen) in den Vorratsbehälter 3 führt.

Schließlich ist zumindest eine Überlaufrohr-Rücklaufleitung 301 vorgesehen, welche vom unteren Ende des Überlaufrohrs 103 in den Vorratsbehälter 3 führt und auch über ein Absperrventil verfügen kann.

Experimentelle Untersuchungen zur Optimierung der Leistung und der Funktion des erfindungsgemäßen Biogasreaktors haben Folgendes ergeben.
1. Zusammensetzung des Reststoffgemisches hinsichtlich dessen Energiepotentials am Beispiel typischer Abfallstoffe in Pferdehof Anlagen.
   Vorherrschende Meinung bei Pferdehofbetreibern ist die, dass Pferdeäpfel allein zu energiearm seien, eine Biogasfermentation in Gang zu halten. Deshalb wird eine Beimischung von Gülle, Hühnermist, Grünschnitt und Kuhmist möglichst aus nahegelegenen landwirtschaftlichen Betrieben sinnvoll sein, womit auch deren im Zuge immer schärfer werdender Gesetzesregelungen immer gravierender und kostspieliger werdender Entsorgungsproblematik wirtschaftlich abgeholfen wird. Die Rücklieferung des in einer Biogasanlage mit dem erfindungsgemäßen Biogasreaktor produzierten hochwertigen Düngers an die nachbarschaftlichen Betriebe bietet sich als regionale synergetische Kooperation an. Je reicher die zugeführte Reststoffmischung an Biogas generierenden Stoffen ist, desto höher wird die vom geometrisch gegebenen Reaktorvolumen zu erwartende Leistung bezüglich Biogas- und Düngerproduktion werden. Der begrenzende Faktor der Beimischung energiespendender Zuschlagstoffe zu dem energieärmeren, von der Anlage zu verarbeitenden Pferdeäpfelsubstrat ist andererseits die von den Pferdehofbetreibern angestrebte Vollverwertung der im eigenen Betrieb anfallenden Abfallstoffe.
2. Einfluss des Trockensubstanzgehaltes der dem Reaktor zugeführten Reststoffmischung auf dessen Kombination aus Durchsatz- und Biogasleistung und "Reinheitsgrad" des am Konusende anfallenden Wertstoffes Dünger.

Allgemeinwissen der Biomechanik sagt, dass Fermentationsprozesse umso effizienter ablaufen, je flüssiger ein Substratgut ist. Bei dem erfindungsgemäßen sehr schlanken und hohen zylindrokonischen Biogasreaktor darf antizipiert werden, dass auch bei hohem Trockensubstanzgehalt im unteren Drittel der Füllhöhe eingespeistes Gemisch aus frischem und in Hochgärung befindlichem Substratgut durch seine heftige Biogas Entbindung im trocken/breiigen darüber befindlichen Substratgut einerseits einen die Fermentation heftig antreibenden "Rühreffekt" bewirkt und andererseits die Sedimentation ausfermentierten Wertstoffes "Dünger" im unteren konischen Teil des Reaktors zulässt.

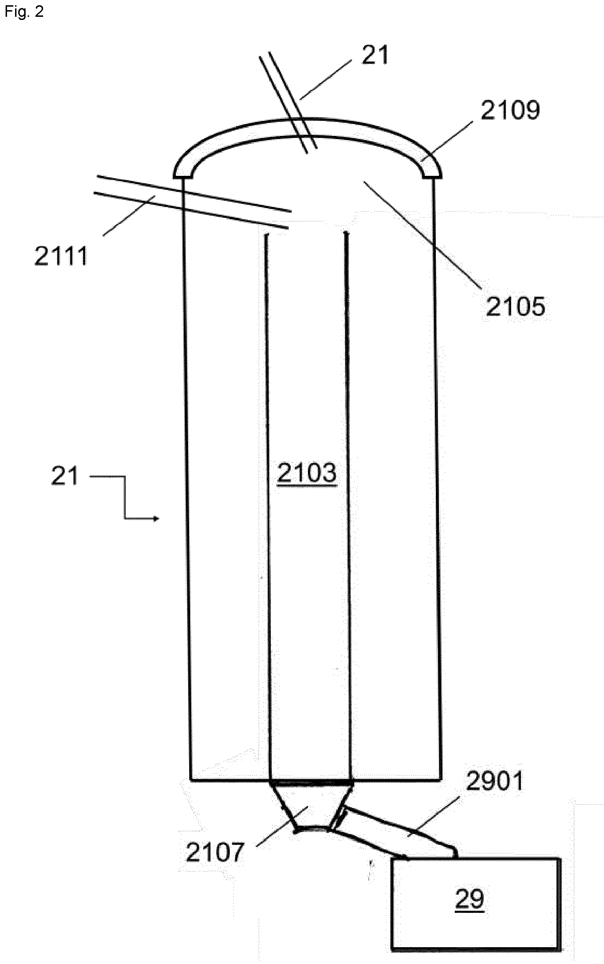

In Figur 2 wird die erfindungsgemäße Endfermentationsstufe in einer bevorzugten Ausführungsform dargestellt. Diese umfasst zunächst einen zylindrischen Behälter 21, der in seinem Innenraum zumindest ein Überlaufrohr 2103 und eine Biogas-Sammelzone 2105 aufweist. Insofern ist die erfindungsgemäße Endfermentationsstufe im Wesentlichen identisch mit dem erfindungsgemäßen Biogasreaktor.

Nach oben wird der zylindrische Behälter 21 mit einem Deckel 2109 abschlossen, an dem oder durch den zumindest eine Zufuhrleitung 2111 führt, die in den Innenraum des zylindrischen Behälters 21 zum oberen Ende des Überlaufrohrs 2103 führt. Ferner ist zumindest eine Biogas-Entnahmeleitung 27 zum Abführen von entstandenem Biogas vorgesehen.

Am unteren Ende ist zumindest ein Wertstoff-Sammelbehälter 29 vorgesehen, der unterhalb des zylindrischen Behälters 21 angeordnet und über eine Wertstoff-Sammelleitung 2901 mit dem Überlaufrohr 2103 verbunden ist. Erfindungswesentlich ist, dass der zylindrische Behälter 21 vertikal angeordnet ist. Dabei schließt "vertikal" auch eine Abweichung von einigen Grad aus der Vertikal ein, solange das Absinken des Substratguts in dem Überlaufrohr 2103 nicht beeinträchtigt wird.

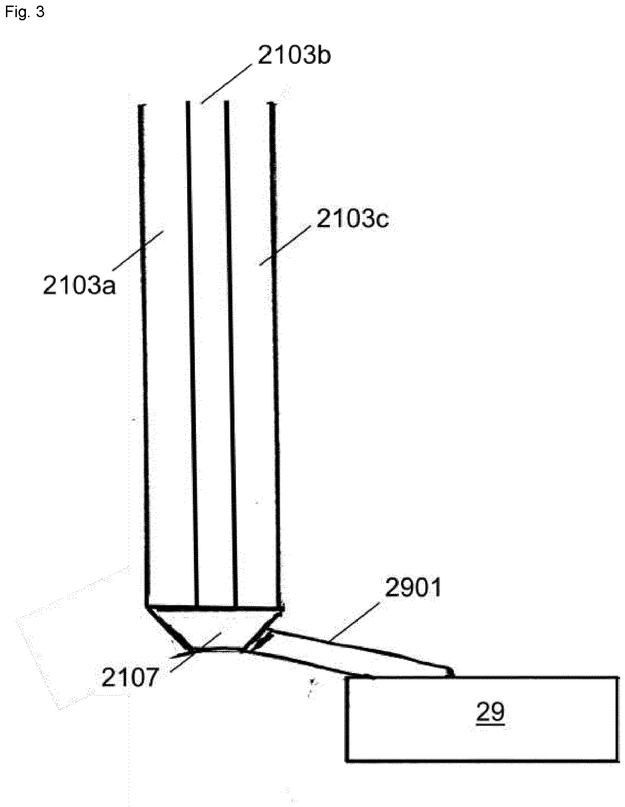

Figur 3 zeigt eine schematische Detailansicht einer erfindungsgemäßen Endfermentationsstufe, bei welcher das Überlaufrohr 2103 in drei Teilzellen 2103a, 2103b, 2103c vertikal unterteilt ist. Am unteren Ende münden alle drei Teilzellen 2103a, 2103b, 2103c in den konischen Auslauf 2107, der über die Sammelleitung 2901 mit dem Wertstoff-Sammelbehälter 29 verbunden ist.

Die vorliegende Erfindung stellt dem Konzept der horizontalen Lagerung von im Wesentlichen ausfermentiertem Substratgut auf einer wasserdichten Fläche mit einer Folienabdeckung das erfindungsgemäße Konzept eines aufrecht stehenden Biogasreaktors und einer aufrecht stehenden Endfermentationsstufe entgegen, was bereits im Flächenbedarf und damit in der Variabilität der Anlagen große Vorteile bringt.

Abhängig vom benötigten Durchsatz können einer oder mehrere erfindungsgemäße Biogasreaktoren bzw. eine oder mehrere erfindungsgemäße Endfermentationsstufen vorgesehen werden, die von oben mit Substratgut aus der Endstufe konventioneller Biogasablagen beschickt werden. Das Substratgut wird im schichtweisen Absinken zum unteren Ende des Überlaufrohrs 103, 2103 vollständig fermentiert und als sofort düngungsgeeignetes und zulässiges Substratgut entnommen.

Das Prinzip "first in - first out" wird erfindungsgemäß durch die Wahl eines Querschnitts des Überlaufrohrs 103, 2103 gewährleistet, was eine vertikale Vermischung des Substratgutes durch eine Art "kochende Gärung" verhindert. Wenn die Wahl einer Vielzahl schlanker Rohre als Überlaufrohre 103, 2103 zu hohe Kosten des Systems verursacht, kann auch ein Behälter 21 mit großem Durchmesser gewählt werden, der im Inneren durch einwandige, kostengünstige Zwischenwände unterteilt ist.

### Bezugszeichenliste

- 1: zylindrischer Druckbehälter
- 101: Fermentationszone
- 103: Überlaufrohr
- 103a: Substratüberlauf
- 105: Biogas-Sammelzone
- 107: konischer Auslauf
- 3: Vorratsbehälter
- 301: Überlaufrohr-Rücklaufleitung
- 5: Rohsubstrat-Zufuhrleitung
- 7: Biogas-Entnahmeleitung
- 9: Wertstoff-Sammelbehälter
- 901: Wertstoff-Sammelleitung
- 11: Zirkulationsleitung
- 13: Reinigungs-Vorlaufleitung
- 15: Reinigungs-Rücklaufleitung
- 17: Biogas-Sammelbehälter
- 21: Behälter
- 2101: Fermentationszone
- 2103: Überlaufrohr
- 2105: Biogas-Sammelzone
- 2107: konischer Auslauf
- 2109: Deckel
- 2111: Zufuhrleitung
- 2103a: Teilzelle
- 2103b: Teilzelle
- 2103c: Teilzelle
- 27: Biogas-Entnahmeleitung
- 29: Sammelbehälter
- 2901: Wertstoff-Sammelleitung

## Patentansprüche

1. Biogasreaktor umfassend
- einen zylindrischen Druckbehälter (1), der in seinem Innenraum zumindest eine Fermentationszone (101), ein Überlaufrohr (103) und eine Biogas-Sammelzone (105) aufweist,
- zumindest einen Vorratsbehälter (3) zur Bevorratung von Rohsubstrat,
- zumindest eine Rohsubstrat-Zufuhrleitung (5), die von dem Vorratsbehälter (3) in die Fermentationszone (101) des zylindrischen Druckbehälters (1) führt,
- zumindest eine Biogas-Entnahmeleitung (7) zum Abführen von entstandenem Biogas und
- zumindest einen Wertstoff-Sammelbehälter (9), der unterhalb des zylindrischen Druckbehälters (1) angeordnet und über eine Wertstoff-Sammelleitung (901) mit dem Überlaufrohr (103) verbunden ist,
wobei das Substratüberlauf (103a) des Überlaufrohrs (103) mindestens in der oberen Hälfte des zylindrischen Druckbehälters (1) positioniert ist.

2. Biogasreaktor nach Anspruch 1, wobei der zylindrische Druckbehälter (1) am unteren Ende des Überlaufrohrs (103) einen konischen Auslauf (107) aufweist, der zumindest mit einer Wertstoff-Sammelleitung (901) in Verbindung steht.

3. Biogasreaktor nach Anspruch 1 oder 2, ferner umfassend eine Zirkulationsleitung (11) zum Zirkulieren von fermentierendem Substrat aus einem oberen Bereich der Fermentationszone (101) in einen unteren Bereich der Fermentationszone (101).

4. Biogasreaktor nach einem der Ansprüche 1 bis 3, ferner umfassend
- eine Reinigungs-Vorlaufleitung (13), welche von der Rohsubstrat-Zufuhrleitung (5) in die Biogas-Sammelzone (105) führt, und
- zumindest eine Reinigungs-Rücklaufleitung (15), welche vom unteren Ende der Fermentationszone (101) in den Vorratsbehälter (3) führt.

5. Biogasreaktor nach einem der Ansprüche 1 bis 4, ferner umfassend zumindest ein Überlaufrohr-Rücklaufleitung (301), welche vom unteren Ende des Überlaufrohrs (103) in den Vorratsbehälter (3) führt.

6. Biogasreaktor nach einem der Ansprüche 1 bis 5, wobei das Verhältnis der Höhe des zylindrischen Druckbehälters (1) zu dessen Durchmesser bei 2 : 1 bis 7 : 1, bevorzugt bei 4 : 1 bis 6 : 1, liegt.

7. Verfahren zum Betreiben eines Biogasreaktors, umfassend die Schritte
a) Erstbefüllen zumindest einer Fermentationszone (101) eines Biogasreaktors nach einem der Ansprüche 1 bis 6 mit einem biologisch beimpften Rohsubstrat,
b) Fermentieren des Rohsubstrats in der Fermentationszone (101) über deren gesamte Höhe,
c) Abführen des ausfermentierten Substratguts über ein Überlaufrohr (103) und Auffangen in einem Wertstoff-Sammelbehälter (9),
d) Nachspeisen der Fermentationszone (101) mit Rohsubstrat in dem Umfang, in dem ausfermentiertes Substratgut abgeführt wird, und
e) Durchführen des weiteren Fermentierens zumindest in der oberen Hälfte der Fermentationszone (101).

8. Verfahren nach Anspruch 7, wobei parallel zu den Schritten b), c) und d) fermentierendes Substrat über eine Zirkulationsleitung (11) aus einem mittleren Bereich der Fermentationszone (101) in einen unteren Bereich der Fermentationszone (101) zirkuliert wird.

9. Verwendung des Biogasreaktors nach einem der Ansprüche 1 bis 6 als Endfermentationsstufe einer konventionellen Biogasanlage.

10. Endfermentationsstufe für Biogasreaktoren und Biogasanlagen umfassend
- einen zylindrischen Behälter (21), der in seinem Innenraum zumindest ein Überlaufrohr (2103) und eine Biogas-Sammelzone (2105) aufweist,
- einen den zylindrischen Behälter (21) nach oben abschließenden Deckel (2109),
- zumindest eine Zufuhrleitung (2111), die in den Innenraum des zylindrischen Behälters (21) zum oberen Ende des Überlaufrohrs (2103) führt,
- zumindest eine Biogas-Entnahmeleitung (27) zum Abführen von entstandenem Biogas und
- zumindest einen Wertstoff-Sammelbehälter (29), der unterhalb des zylindrischen Behälters (21) angeordnet und über eine Wertstoff-Sammelleitung (...) mit dem Überlaufrohr (2103) verbunden ist,
wobei der zylindrische Behälter (21) vertikal angeordnet ist.

11. Endfermentationsstufe nach Anspruch 10, wobei der zylindrische Behälter (21) am unteren Ende des Überlaufrohrs (2103) einen konischen Auslauf (2107) aufweist, der zumindest mit einer Wertstoff-Sammelleitung (2901) in Verbindung steht.

12. Endfermentationsstufe nach Anspruch 10 oder 11, wobei das Überlaufrohr (2103) in vertikaler Richtung in zumindest zwei Teilzellen (2103a, 2103b) unterteilt ist.

13. Endfermentationsstufe nach einem der Ansprüche 10 bis 12, wobei am Eintritt in den zylindrischen Behälter (21) ein Durchlaufheizregister zur Temperierung von fermentierendem Substrat vorgesehen ist.

14. Endfermentationsstufe nach einem der Ansprüche 10 bis 13, wobei in dem Überlaufrohr (2103) katalytische Elemente vorgesehen sind.

15. Endfermentationsstufe nach einem der Ansprüche 10 bis 14, wobei das Verhältnis der Höhe des Überlaufrohrs (2103) oder der Höhe jedes der zumindest zwei Teilzellen (2103.1, 2103.2) zu deren Durchmesser bei 2 : 1 bis 7 : 1 liegt.
